# EUROPEAN PATENT SPECIFICATION

(11) **EP 1 394 261 B1**
(45) Date of publication and mention of the grant of the patent: **30.03.2011**
(21) Application number: 02739002.0
(22) Date of filing: 18.04.2002
(51) Int. Cl.: A61K 39/42, C07K 14/16, C07K 14/47, C07K 16/10, C07K 19/00, C12N 9/00, C12N 15/62, C12P 21/08

(54) **ANTIGENS FOR IMMUNISATION**
ANTIGENE ZUR IMMUNISIERUNG
ANTICORPS POUR IMMUNISATION

(30) Priority: 24.04.2001 RU 2001110759
(43) Date of publication of application: 03.03.2004
(60) Divisional application: 10183175.8
(73) Proprietor: FDS Pharma Ass., London E1 8NN (GB)
(72) Inventor: GABIBOV, Alexandr Gabibovich, Moscow, 109028 (RU); KOLESNIKOV, Alexandr, Vladimirovich, Moscow, 117279 (RU); PONOMARENKO, Natalya Alexandrovna, Moscow, 117333 (RU); ALEXANDROVA, Elena Sergeevna, Moscow, 117312 (RU); VOROBIEV, Ivan Ivanovich, Moscow, 119423 (RU); DEMIN, Alexandr Viktorovich, Noginskyraion, Moskovskaya obl., 142432 (RU)
(74) Representative: Helino, Timo Kalervo
(86) International application number: PCT/RU2002/000177
(87) International publication number: WO 2002/086058

(56) References cited:
- WO-A-99/48925
- WO-A1-97/03696
- RU-A1- 94 045 907
- US-A- 5 792 459
- US-A- 5 948 764
- US-A- 5 961 973
- US-A- 6 140 091
- KARLE S ET AL: "Characterization of antibodies elicited by a conserved gp120 peptide" FASEB JOURNAL, FED. OF AMERICAN SOC. FOR EXPERIMENTAL BIOLOGY, BETHESDA, MD, US, vol. 15, no. 5, 8 March 2001 (2001-03-08), page A1189, XP008087935 ISSN: 0892-6638
- SAKAI K ET AL: "Characterization of a major encephalitogenic T cell epitope in SJL/J mice with synthetic oligopeptides of myelin basic protein." JOURNAL OF NEUROIMMUNOLOGY AUG 1988, vol. 19, no. 1-2, August 1988 (1988-08), pages 21-32, XP002467027 ISSN: 0165-5728
- FRANZIANO M ET AL: "EPITOPE SPECIFICITY OF ANTI-HIV ANTIBODIES IN HUMAN AND MURINE AUTOIMMUNE DISEASES" AIDS RESEARCH AND HUMAN RETROVIRUSES, MARY ANN LIEBERT, US, vol. 12, no. 6, 1996, pages 491-496, XP008070550 ISSN: 0889-2229
- ZHOU Y -X ET AL: "Binding of native EGFR and interference with EGFR function by antibodies to a linear EGFR peptide" FASEB JOURNAL, vol. 15, no. 5, 8 March 2001 (2001-03-08), page A1199, XP001033976 & ANNUAL MEETING OF THE FEDERATION OF AMERICAN SOCIETIES FOR EXPERIMENTAL BIOLOGY ON EXPERIMENTAL BIOL; ORLANDO, FLORIDA, USA; MARCH 31-APRIL 04, 2001 ISSN: 0892-6638
- TAWFIK D S ET AL: "Unexpectedly high occurrence of catalytic antibodies in MRL/lpr and SJL mice immunized with a transition-state analog: is there a linkage to autoimmunity?" PROCEEDINGS OF THE NATIONAL ACADEMY OF SCIENCES OF THE UNITED STATES OF AMERICA 14 MAR 1995, vol. 92, no. 6, 14 March 1995 (1995-03-14), pages 2145-2149, XP002467028 ISSN: 0027-8424
- PONOMARENKO N A ET AL: "Natural antibody catalytic activities in mice with autoimmune disorders" DOKLADY BIOCHEMISTRY, PLENUM PUBLISHING PRESS, NEW YORK, NY, US, vol. 375, November 2000 (2000-11), pages 224-227, XP008087911 ISSN: 0012-4958
- TAKAHASHI N ET AL: "Improved generation of catalytic antibodies by MRL/MPJ-lpr/lpr autoimmune mice" JOURNAL OF IMMUNOLOGICAL METHODS, ELSEVIER SCIENCE PUBLISHERS B.V.,AMSTERDAM, NL, vol. 235, no. 1-2, February 2000 (2000-02), pages 113-120, XP004188237 ISSN: 0022-1759
- PONOMARENKO NATALIA A ET AL: "Induction of a protein-targeted catalytic response in autoimmune prone mice: antibody-mediated cleavage of HIV-1 glycoprotein GP120." BIOCHEMISTRY 10 JAN 2006, vol. 45, no. 1, 10 January 2006 (2006-01-10), pages 324-330, XP002467029 ISSN: 0006-2960
- GABIBOV A G ET AL: "Antibody proteases: Induction of catalytic response" BIOCHEMISTRY, CONSULTANTS BUREAU, NEW YORK, NY, US, vol. 67, no. 10, October 2002 (2002-10), pages 1168-1179, XP008087943 ISSN: 0006-2979

<110> "ASGL - Farmatsevticheskie Innovatsii", zakrytoe aktsionernoe obschestvo
<120> Method for producing catalytic antibodies (variants), antigens for immunization and nucleotide sequence
<140> 2001110759/13 (011749)
   <141> 24.04.2001
<160> 2
   <210> 1
   <211> 365
   <212> PTR
   <213> Artificial Sequence
   <400> 1
<210> 2
   <211> 1149
   <212> DNA
   <213> Artificial Sequence
<400> 2
   ATGGCTACAG   AAAAATTGTG   GGTCACAGTC   TATTATGGGG   TACCTGTGTG   GAAGGAAGCA     60
   ACCACCACTC   TATTTTGTGC   ATCAGATGCT   AAAGCATATG   ATACAGAGGT   ACATAATGTT    120
   TGGGCCACAC   ATGCCTGTGT   ACCCACAGAC   CCCAACGAAG   TAGTATTGAG   CTGCAACACC    180
   TCTGTCATTA CACAGGCCTG TCCAAAGGTA TCCTTTGAGC CAATTCCCAT ACATTATTGT  240
   GCCCCGGCTG GTTTTGCGAT TCTAAAATGT AATAATAAGA CGTTCAATGG AACAGGACCA  300
   TGTACAAATG   TCAGCACAGT   ACAATGTACA   CATGGAATTA   GGCCAGTAGT   ATCAACTCAA    360
   CTGCTGTTAA   ATGGCAGTCT   AGCAGAAGAA   GAGGTAGTAA   TTAGATCTGT   CAATTTCACG    420
   GACAATGCTA   AAACCATAAT   AGTACAGCTG   AACACATCTG   TAGAAATTAA   TTGTACACAT    480
   TGTAACATTA   GTAGAGCAAA   ATGGAATAAC   ACTTTAAAAC   AGATAGCTAG   CAAATTAAGA    540
   GAACAATTTG   GAAATAATAA   AACAATAATC   TTTAAGCAAT   CCTCAGGAGG   GGACCCAGAA    600
   ATTGTAACGC   ACAGTTTTAA   TTGTGGAGGG   GAATTTTTCT   ACTGTAATTC   AACACAACTG    660
   TTTAATAGTA   CTTGGTTTAA   TAGTACTTGG   AGTACTGAAG   GGTCAAATAA   CACTGAAGGA    720
   AGTGACACAA   TCACCCTCCC   ATGCAGAATA   AAACAAATTA   TAAACATGTG   GCAGAAAGTA    780
   GGAAAAGCAA   TGTATGCCCC   TCCCATCAGT   GGACAAATTA   GATGTTCATC   AAATATTACA    840
   GGGCTGCTAT   TAACAAGAGA   TGGTGGTAAT   AGCAACAATG   AGTCCGAGAT   CTTCAGACCT    900
   GGAGGAGGAG   ATATGAGGGA   CAATTGGAGA   AGTGAATTAT   ATAAATATAA   AGTAGTAAAA    960
   ATTGAACCAT   TAGGAGTAGC   ACCCACCAAG   GCAAAGCTGG   ATCCCCACCA   CCACCACCAC   1020
   CACGGTTCCG   GCCAACAAAA   ACTCATCTCA   GAAGAGGATC   TGAATTCGAG   CTCCGTCGAC   1080
   AAGCTTGCGG   CCGCAGTAGT   CCATTTCTTC   AAGAACATTG   TGACACCTCG   AACACCACCT   1140
   CCATCCTAA                                                                  1149

## Description

### Field of the invention

The present invention relates to biotechnology, immunology, genetic engineering, the microbiological and medicinal industries and antigens for immunization. It comprises a combined approach to the manufacture and expression of catalytically active antibodies which are potential therapeuticals intended to destroy protein antigens, in particular gp120, which is the main surface protein of human immunodeficiency virus.

### Prior art

It is known that catalytic antibodies targeted to physiologically active substances and natural objects useful in biomedicine may be designed as specific representations of transition states of modeled chemical conversions. US Patent No. 5,948,658 discloses an antibody designed by the above approach and capable of specifically cleavaging narcotic cocaine. In spite of a highly developed technology for the production of monoclonal antibodies, this approach cannot be effective in the case of high molecular biopolymers, proteins, and peptides because it is difficult to model corresponding transition states of the reaction.

The production of catalytic antibodies directly active against gp120 is disclosed in WO 9703696; however, according to WO 9703696, the antibody is obtained from patient serum, which impedes development of a unified medical technology for medical drug production.

Karle et. al. "Characterization of antibodies elicited by a conserved gb120 peptide" FASEB JOURNAL, FED. OF AMERICAN SOC. FOR EXPERIMENTAL BIOLOGY, BETHESDA, MD, US, vol. 15, no. 5, disclose antibodies raised to a conserved gp120 epitope (421-436) in the autoimmune MRL/lpr mice.

### Summary of the Invention

By developing a method for producing catalytic antibodies against proteins and peptides, in particular gp120, with the use of animals with spontaneous and inducible autoimmune pathologies, it is possible to design a "catalytic vaccine" which, upon injection to a patient, is capable not only of binding the antigen but also of destroying it thus inhibiting the development of disease.

Described is a method for producing catalytic antibodies which uses animals with spontaneous and induces autoimmune pathologies. Mice are used as the animals with spontaneous and inducible autoimmune pathologies. The used mice belong to strains for which immunization with myelin basic protein or its fragment can induce the development of experimental autoimmune encephalomyelitis. The animals are administered with a fusion protein consisting of myelin basic protein or its fragments and a potential substrate of catalytic antibody or a fragment of the potential substrate. The potential substrate is gp120 (surface glycoprotein of HIV-1) or its fragments.

The object of the present invention is the fusion protein having the following structure:

A nucleotide sequence encoding the fusion protein of the above structure is proposed.

A method for producing catalytic antibody uses animals with spontaneous and induces autoimmune pathologies is described. The animal with spontaneous and inducible autoimmune pathologies are MRL-lpr/lpr mice, the mice being immunized with an antigen containing a haptene, the hapten being a conjugate of a mechanism-dependent covalent protease inhibitor with a peptide, the peptide being a fragment of a potential substrate of the catalytic antibody.

The potential substrate of the catalytic antibody is gp120 or its fragment is described.

Described is peptidylphosphonate used in the method which has the following structure:

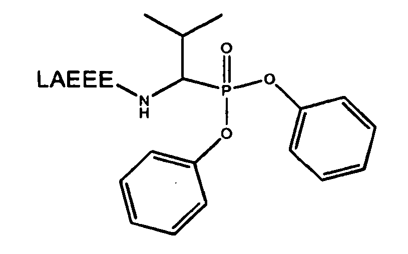

### Brief Description of the Drawings:

**FIG. 1****.** Nucleotide sequence of **gp120 fragment I-III**. Colored are fragments I, II and III, sequentially. The primers used in PCR are marked out with capital letters.
**FIG. 2****.** Diagrams of recombinant proteins obtained with the use of pET32b vector.
**FIG. 3****.** Diagrams of recombinant proteins obtained with the use of pET28a vector.

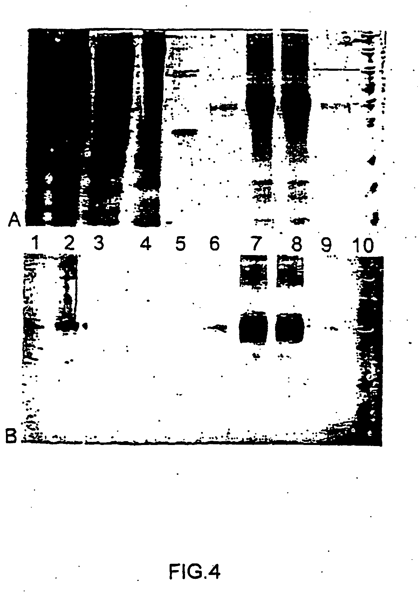

**FIG. 4****.** Electrophoregram (A) and immunoblot (B) of different stages of isolation and purification of the protein **gp120I-IIImbp** (the product of construct 15). 1 - total cellular proteins before induction; 2 - total cellular proteins after induction; 3 - the fraction of soluble intracellular proteins; 4 - soluble proteins not retained by the metal chelate column; 5 - soluble proteins eluted at pH 5.0; 6 - soluble protein form preparation after chromatographic purification; 7 - the fraction of insoluble intracellular proteins; 8 - insoluble proteins not retained by metal chelate column; 9 - denaturated protein form preparation after chromatographic purification; 10 - marker.
**FIG. 5****.** Analysis of the antigen specificity of antibodies in serum of SJL mice immunized with the fusion protein **gp120I-IIImbp** at different doses. The figure legend indicates the immunogen dose; ***SJL-2*** are mice immunized with the dose of 150 µg per mouse, ***SJL-3*** are mice immunized with the dose of 300 µg per mouse.
**FIG. 6****.** The principles of fluorescent and enzymic analyses of proteolytic activity.

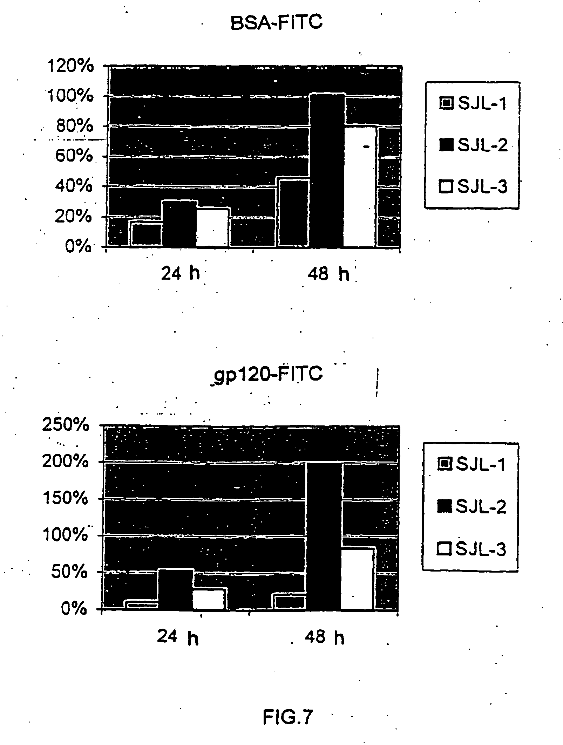

**FIG. 7****.** Determination of the proteolytic activity of antibody preparation isolated from serum of SJL mice immunized with the fusion protein **gp120I-IIImbp. *SJL-1*** are control mice. ***SJL-2*** are mice immunized with the dose of 150 µg per mouse. ***SJL-3*** are mice immunized with the dose of 300 µg per mouse. BSA-FITC and gp120-FITC were used as substrates.

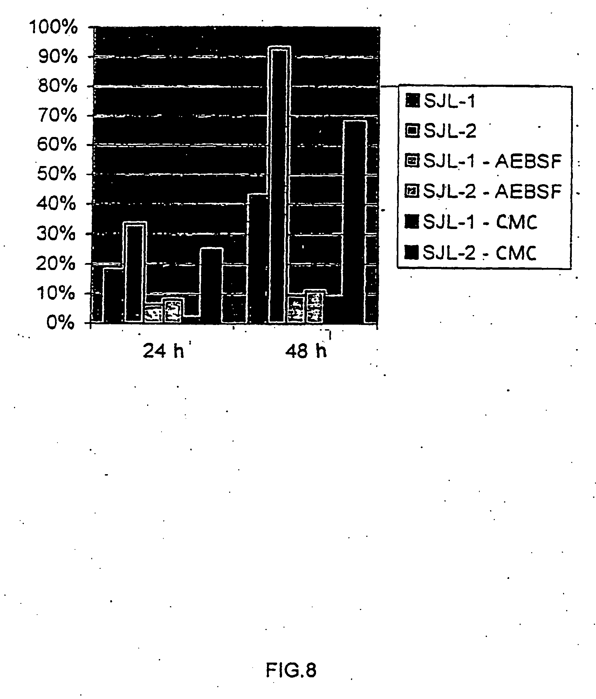

**FIG. 8****.** Inhibition of the proteolytic activity of antibody preparation isolated from serum of
   ***SJL*** mice immunized with the fusion protein **gp120I-IIImbp. *SJL-1*** are control mice.
   ***SJL-2*** are mice immunized with the dose of 150 µg per mouse.
   AEBSF: aminoethanebenzenesulfonyl fluoride.
   CMC: phenylalanylchloromethylketone.

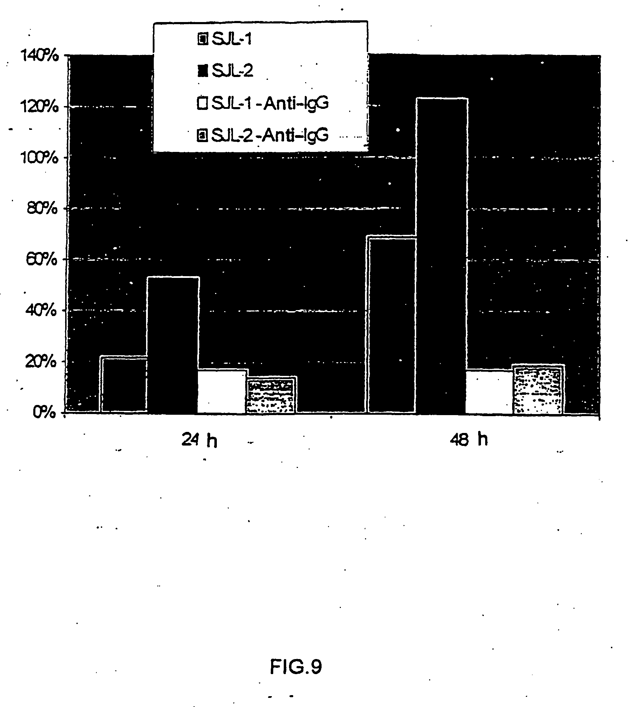

**FIG. 9****.** Antispecies antibody inhibition of the proteolytic activity of antibody preparation isolated from serum of SJL mice immunized with **gp120I-IIImbp. *SJL-1*** are control mice. ***SJL-2*** are mice immunized with the dose of 150 µg per mouse. Anti-IgG: rabbit polyclonal antibodies against murine IgG.
**FIG. 10****.** Enzymatic determination of the proteolytic activity of antibody preparations isolated from sera *of SJL* mice immunized with the fusion proteins **gp120I-IIImbp** at different doses. A: ***SJL-1*** are control mice; ***SJL-2*** are mice immunized with the dose of 150 µg per mouse; ***SJL-3*** are mice immunized with the dose of 300 µg per mouse; ***CBA*** are control CBA mice.

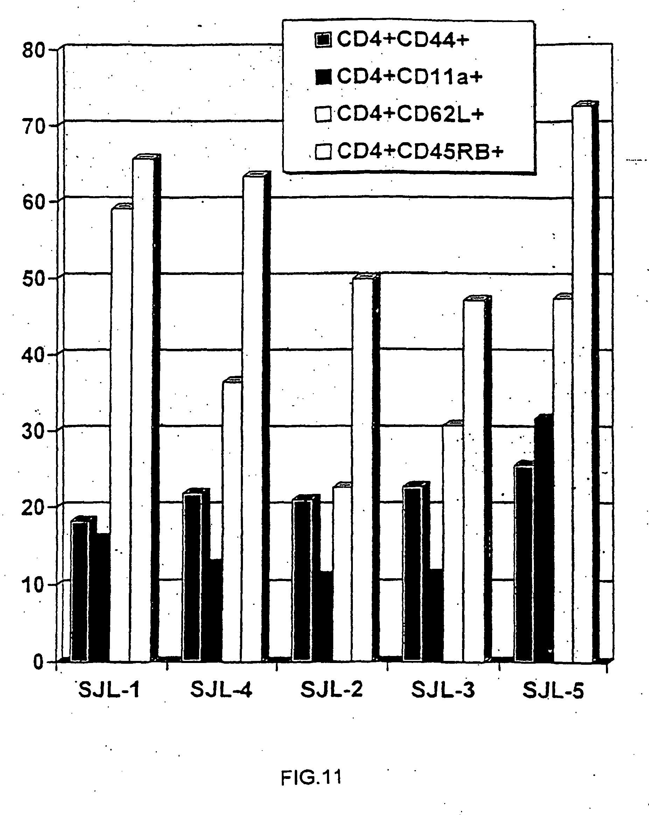

**FIG. 11****.** Changes in the expression level of surface markers of T-cells of the immune system of SJL mice immunized with the fusion protein **gp120I-IIImbp** at different doses, with the recombinant protein **gp120I-III,** and with the encephalitogenic peptide MBP₈₉₋₁₀₄. ***SJL-1*** are non-immunized mice. ***SJL-2*** are mice immunized with **gp120I-IIImbp** at the dose of 150 µg per mouse. ***SJL-3**:* mice immunized with **gp120I-IIImbp** at the dose of 300 µg per mouse. ***SJL-4*** are mice immunized with the peptide **MBP₈₉₋₁₀₄**. ***SJL-5*** are mice immunized with the recombinant protein **gp120I-III** at the dose of 300 µg per mouse.
**FIG. 12****.** Enzyme immunoassay of sera of ***SJL, MRL-lpr*/*****lpr*** and ***NZB*/*NZW F1*** mice immunized with peptidylphosphonate. The antigen used was: A - biotinylated reactive peptide; B - biotinylated diphenylvalylphosphonate; C - methyl p-nitrophenyl biotinylphenylmethylphosphonate.
**FIG. 13****.** Electrophoregram (A) and immunoblot (B) of polyclonal antibodies isolated from immunized mice of strains ***SJL*** (4), ***MRL-lprllpr*** (5) and ***NZB*/*NZW F1*** (6) and covalently modified with an antigen. Lanes 1-3: 10µg of BSA, 1 µg of trypsin, and 1 µg of IgG of BALB/c mice.

The present invention is illustrated by the following Examples:

### Example 1. Development of a genetic construct containing a nucleotide sequence encoding the fusion protein gp120I-IIImbp for its expression in a prokaryotic system.

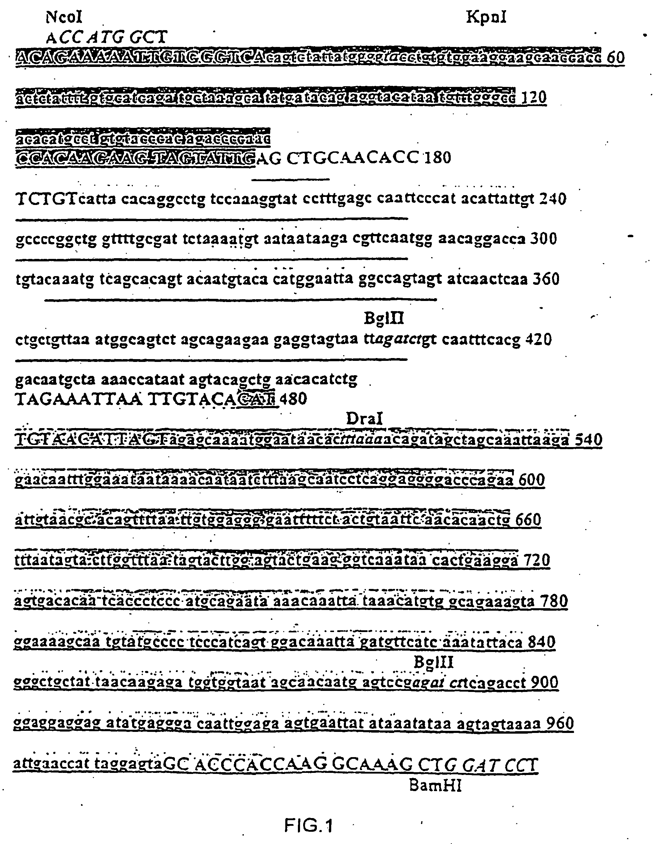

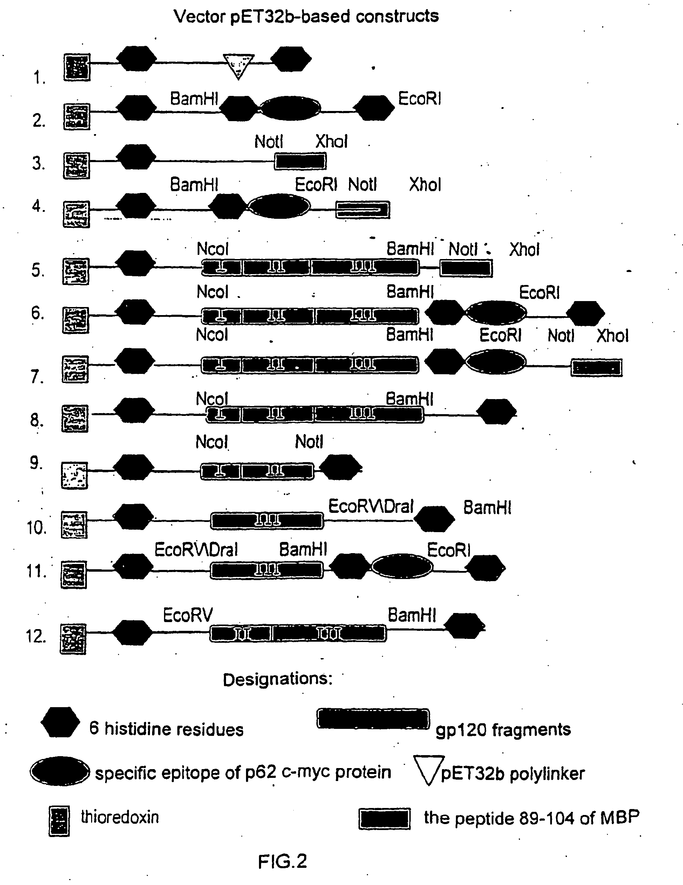

1) To induce autoimmune encephalomyelitis (EAE) in ***SJL*** mice, the 89-104 peptide of the myelin basic protein (MBP) was chosen, the peptide having the following composition: **VVHFFKNIVTPRTPPPS** [Sakai, K., Zamvil, S.S., Mitchell, D.J., Lim, M., Rothbard, J.B., and Steinman, L. 1988. Characterization of a major encephalitogenic T cell epitope in SJL/J mice with synthetic oligopeptides of myelin basic protein. J. Neuroimmunol. 19:21-32., || Tan, L.J., Kennedy, M.K., and Miller, S.D. 1992. Regulation of the effector stages of experimental autoimmune encephalomyelitis via neuroantigen-specific tolerance induction. II. Fine specificity of effector T cell inhibition. J. Immunol. 148:2748-2755.]. The DNA sequence corresponding to said peptide was synthesized by PCR from two overlapping oligonucleotides additionally containing a stop codon and restriction sites. The resulting DNA fragment was cloned in the pET32b plasmid using NotI and XhoI restrictases. The resulting plasmid is hereinafter designated as **pET32mbp**. For the accurate identification of recombinant proteins at all stages of their expression, isolation and purification, **pET32bCH** and **pET32CHmbp** constructs were engineered to contain a sequence that codes for the 10 amino acid-long immunodominant epitope of human **p62 c-myc** protein [Evan G.I., Lewis G.K., Ramsay G., Bishop J.M.. ll Mol. Cell. Biol. 1985, V.5(12), P. 3610-3616.].
2) Numerous available publications on the structure, immunogenicity and functional activity of the surface protein gp120 [Hansen, J.E., Lund, O., Nielsen, J.O., Brunak, S., and Hansen, J.-E., S. 1996. Prediction of the secondary structure of HIV-1 gp120. Proteins. 25: 1-11 || Shioda, T., Oka, S., Xin, X., Liu, H., Harukuni, R., Kurotani, A., Fukushima, M., Hasan, M.K., Shiino, T., Takebe, Y., Iwamoto, A. and Nagai, Y. 1997. In vivo sequence variability of human immunodeficiency virus type I envelope gp 120: association of V2 extension with slow disease progression. J. Virol. 71: 4871-4881 Sullivan, N., Sun, Y., Sattentau, Q., Thali, M., Wu, D., Denisova, G., Gershoni, J., Robinson, J., Moore, J., and Sodroski, J. 1998. CD4-Induced Conformational Changes in the Human Immunodeficiency Virus Type 1 gp120 Glycoprotein: Consequences for Virus Entry and Neutralization. J. Virol. 72: 4694-4703] allowed allocation of protein regions having a relatively constant sequence and most promising with regard to immunization. For further work, a chimeric polypeptide was chosen, which consisted of three fragments of **gp120** (designated as **I, II** and **III)** lacking the first, second and third hypervariable regions. **HXB2-env** gene sequence was used as the initial template for the synthesis of this construct [Page, K.A., Landau, N.R., and Littman, D.R. 1990. Construction and use of a human immunodeficiency virus vector for analysis of virus infectivity. J. Virol. 64: 5270-5276]. Fragments I, II and III were obtained by PCR using synthetic oligonucleotides followed by assemblage of the fragments using the «splicing by overlap extension» approach (FIG. 1). The final PCR product **I-III** and intermediate products **I-II, II-III** and **III** were cloned into the **BlueScript** plasmid, with subsequent recloning into the plasmids **pET32b** (FIG. 2: No. 8, 9, 10 and 12), **pET32mbp** (FIG. 2: No. 5), **pET32bCH** (FIG. 2: No. 6 and 11) and **pET32CHmbp** (FIG. 2: No. 7) using respective restrictases, i.e., NcoI-BamHI for **I-III,** NcoI-NotI for **I-II,** EcoRV.-BamHI for **II-III,** and EcoRV\DraI.-BamHI for **III.** The products of these constructs were used to test the proteolytic activity of the antibodies against gp120 that had been obtained as a result of immunization.

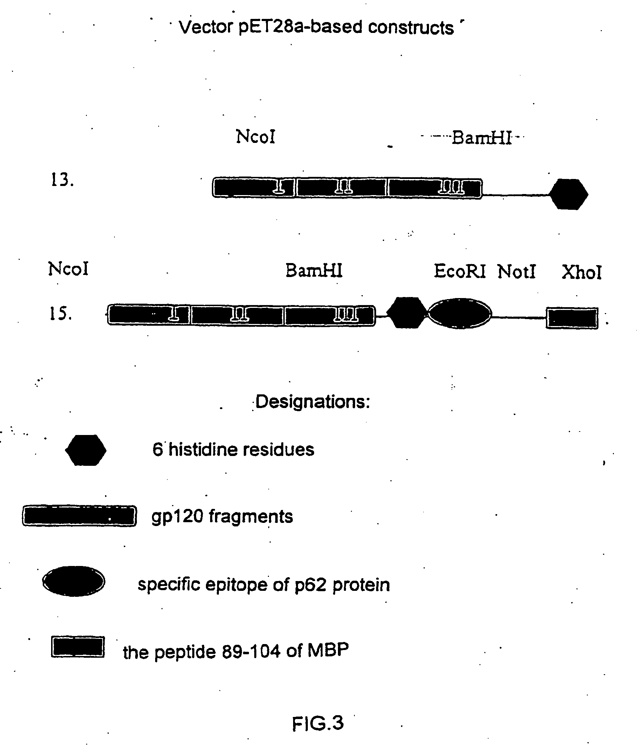

For immunization of ***SJL*** mice, in order to obtain proteolytic antibodies against gyp 120 glycoprotein, the final construct based on **pET28a** vector was engineered (FIG. 3: No. 15). The fragment NcoI-XhoI from the construct 7 were recloned into pET28a at the respective restriction sites. For immune response testing, an additional construct basing on pET28a vector (FIG. 3: No 13) and comprising the gene of gp 120I-III but no sequences encoding mbp peptide and the epitope of c-myc was obtained in a similar way.

The preparation of electrocompetent cells, transformation, restrictase treatment, ligation, PCR, and DNA electrophoresis were carried out according to standard methods [Sambrook J., Fritsch E.F., Maniatis T. // Molecular Cloning: A Laboratory Manual. New York, Cold Spring Harbor Laboratory Press, 1989.].

### Example 2. Expression, isolation and purification of the fusion protein gp120I-III-mbp.

The fusion protein was expressed in T7-lysogenated *E*. *coli* cells (the strain BL21 (DE3) was used in the present example). The protein was expressed as follows:
1. Competent cells are transformed with 0.1 µg of plasmid according to Item 3 of Example 1 by electroporation and seeded onto a Petri dish containing 30 µg/ml Kanamycin and 2% glucose. Bacterial colonies are grown for 12-14 h at 30°C.
2. The colonies are completely suspended in 11 of bacterial medium 2xYT containing 30 mg/ml Kanamycin and 0.1% glucose.
3. The cell culture is grown at 30°C under adequate aeration up to the density of 0.6-1 OU but not longer than three hours; then IPTG is added to make 1 mM and induction is carried out for 3 h at 30°C.

### Fusion protein isolation and purification

The fusion protein gp120I-III-mbp was isolated under denaturating conditions as follows:
1. The cell culture is centrifuged at room temperature for 10 min at 5000 rpm; the sediment is suspended in 1/50 of the initial volume in 50 mM Tris-HCl, pH 8.0; lysozyme and Triton-X100 are added to make 0.1 mg/ml and 0.1%, respectively; and the mixture is incubated for 30 min at 30°C.
2. The lysate is cooled to 0°C, sonicated up to the disappearance of viscosity, and centrifuged for 40 min at 20000 rpm.
3. The sediment is carefully suspended in 1/200 of the initial volume in a buffer containing 50 mM Tris-HCl (pH 8.0), 1 mM EDTA-Na, and 1% Nonidet P-40, centrifuged for 40 min at 20000 rpm, resuspended in chromatographic Buffer A and centrifuged under the same conditions.
4. The sediment is suspended in chromatographic Buffer A (50 mM NaH₂PO₄-Na₂HPO₄, 300 mM NaCl, and 6M urea, pH 8.0), incubated on ice for 1 h, and centrifuged for 10 min at 20000 rpm.
5. The supernatant is applied to a metal chelate column equilibrated with Buffer A at the rate of 10 column volumes per hour, and the column is washed with Buffer B (50 mM NaH₂PO₄-Na₂HPO₄, 300 mM NaCl, and 6 M urea, pH 7.0) at the rate of 30 column volumes per hour up to the discontinuation of baseline migration.
6. Elution is carried out with Buffer B (50 mM NaH₂PO₄-Na₂HPO₄, 20 mM MES-NaOH, 300 mM NaCl, and 6 M urea, pH 5.0) at the rate of 30 column volumes per hour, after which the column is equilibrated with Buffer A for the second time, and immobilized metal ions and retained proteins are removed with Buffer A containing 100 mM EDTA, pH 8.0.
7. The eluate fractions are analyzed by gel electrophoresis and combined, and proteins are precipitated by dialysis against deionized water at room temperature.
8. The protein precipitate is separated by centrifugation at 4000 rpm for 10 min, washed with 70% ethanol, suspended in a minimal volume of 70% ethanol, sonicated up to the discontinuation of particle sedimentation, and stored as suspension at +4°C in sterile polypropylene tubes.

### Fusion protein analysis

To confirm the identity and purity of the resulting preparations of the fusion protein gp 120I-IIImbp, the following characteristics of the protein were determined:
1. The electrophoretic purity of the protein was determined by Method 1 and was found to be 97%.
2. The immunoreactivity with antibodies against c-myc epitope was determined by Method 2, and the protein was found to be immunoreactive.
3. The molecular weight (Da) by mass spectrometry was determined by Method 3 and was found to be 42307 Da, the calculated value being 42075 at the tolerated error of ±2.5%.
4. The specific sorption of the protein (%) by metal chelate sorbent was determined by Method 4 and was found to be >95%.

### Analytical methods:

### 1. Electrophoregam densitometry.

Denaturing electrophoresis of proteins was carried out according to Laemmli using 6 M urea solution in the concentrating and fractionating gels. Gel staining was carried out with Cumassie blue R-250 using contrast enhancing with a cuprum salt. Densitometry was performed with a densitometer or computer assisted plate scanner, with subsequent electrophoregram digitalization and analysis (FIG. 4A).
1. Two-component gel is prepared to have the following composition:
   Upper gel: 6.66% of acrylamide/bis-acrylamide at a 29/l ratio, 0.1% sodium dodecyl sulphate, 0.125 M Tris-HCl, and 6 M urea, pH 6.8.
   Lower gel: 10% of acrylamide/bis-acrylamide at a 29/1 ratio, 0.1% sodium dodecyl sulphate, 0.375 M Tris-HCl and 6 M urea, pH 8.9.
2. Protein samples are mixed with sample buffer containing 5% 2-mecaptoethanol, heated for 5 min at 100°C and applied to gels. Electrophoresis is carried out at 25 mA until indicator dye is eluted.
3. The fractionating gel is separated and incubated for 5 min in a hot mixture of 10% ethanol and 10% acetic acid.
4. Staining is performed by gel incubation for 10 min in a hot mixture of the following composition: 15% ethanol, 25% acetic acid, 0.3 g/l Cumassie Blue R-250, and 0.45 g/l cuprum sulphate hexahydrate.
5. After staining, the gel is subjected to multiple washings, as described in Item 3, up to complete decoloration.
6. The gel is subjected to densitometry according to the densitometer specifications. Upon electrophoregram digitization with a computer-assisted plate scanner, the Green channel of color image or green light filter of the scanner is used. The electrophoregram image is analyzed using Scion Image software by Surface Plot method. The preparation purity is defined as the ratio of the main peak to the sum of all the detected peaks.

### 2. Immunoblotting.

Immunoblotting is carried out accor>ding to the standard regimen using blocking BSA solution. The hybridization buffer is supplemented with bovine serum albumin (fraction V) to make 0.5% (FIG. 4C).
1. Electrophoresis is carried out according to Method 2 using a prestaining marker.
2. The fractionating gel is separated, whereupon the.procedure of transference to HyBond N+ membrane (Amersham) is performed using an LKB apparatus for semidry electrotransference according to the manufacturer's specifications for 40 min at 0.8 mA/cm².
3. The membrane is blocked for 1 h with the solution of 50 mM Tris-HCl (pH 7.6), 150 mM NaCl and 5% bovine serum albumin (fraction V).
4. The membrane is washed thrice for 5 min with a deblocking solution containing 50 mM Tris-HCl (pH 7.6), 150 mM NaCl and 0.05% Tween-20. Then hybridization with the monoclonal antibody 1-9E10.2 is performed for 1 h in the solution of 50 Tris-HCl (pH 7.6), 150 mM NaCl and 0.5% bovine serum albumin.
5. Deblocking (washing) according to Item 4 is performed, and the membrane is hybridized with secondary rabbit Fc-specific anti-mouse IgG antibodies conjugated to horse radish peroxidase (Sigma Immunochemicals) under the same conditions as described in Item 4.
6. The membrane is deblocked as described in Item 4 and stained with the solution of 50 mM Tris-HCl (pH 7.6), 3 mg/ml 1-chloro-4-naphthol and 0.003% H₂O₂ for 30 min.

All the analyses are performed using primary and secondary antibody titers of 1:10000 and 1:4000, respectively, as determined with a characterized antigen, and a test protein is applied to electrophoresis at the dose of 0.1 µg. The presence of a possible test protein immunoreactivity is determined visually by the following criteria: the development of a single distinct well outlined staining zone whose electrophoretic mobility corresponds to that of the test protein. When these criteria are met, instrumental analysis is performed.

For the final semiquantitative analysis, the densitometric evaluation of the intensity of the staining zone is performed by the Surface Plot method using Scion Image software. Test results are considered positive when the peak half-height is 5 times greater than the range of baseline fluctuations on the densitogram.

### 3. MALDI mass-spectrometry.

Samples are prepared for analysis as follows.
1. An aliquot of protein suspension of a minimal volume is evaporated to dryness in a vacuum centrifuge.
2. The residue is dissolved in 1-5 µl of mixture of 1% aqueous trifluoroacetic acid and 30% acetonitrile, applied to the base plate using 2,5-dihydroxybenzoic (DHB) acid as a matrix, and analyzed.
3. A TOF MALDI mass-spectrometer, similar in performance to the VISION 2000 apparatus, is precalibrated by protein reference standards (trypsin and angiotensin), and protein mass-spectra are read using internal calibration. The masses of molecular ions are determined using VISION 2000 Mass Analyzer software taking account of the performed calibrations.

### 4. Specific adsorption.

To confirm the functional properties of a protein preparation, it is tested qualitatively for adsorption from solution by excess metal chelate sorbent. Tested proteins having the sequence 6xHis will be immobilized by the metal chelate sorbent at pH 8.0.
1. The required volume of the metal chelate sorbent Talon (Clontech Laboratories Inc.) is equilibrated with a buffer solution (50 mM Na₂HPO₄-NaH₂PO₄, 300 mM NaCl, and 0.1 % Triton X-100) and 20 µl portions of the 1:1 suspension are transferred to test tubes.
2. 10 µg of test protein solution is added, and the volume is adjusted to 100 µl with the buffer according to Item 1. The test tubes are incubated at shaking for 15 min and allowed to stand, after which 10 µl aliquots are taken for analysis.
3. Adsorption is considered to be complete if the measured protein concentration in the test sample does not exceed 0.005 µg/ml (i.e., is not significantly different from the control when the protein concentration is determined by the BSA test), which corresponds to the 95% absorption level.

### Example 3. Immunization of autoimmune SJL mice with the fusion protein gp120I-IIImbp.

***SJL*** mice are immunized with the fusion protein **gp120 I-III-mbp** as follows.
1. Five female ***SJL*** mice aged 6-8 weeks are immunized twice at a weekly interval with the antigen in complete Freund adjuvant having the final *M. tuberculosis* concentration of 2 mg/ml and the antigen concentrations of 1.5 mg/ml and 3 mg/ml.
2. Injections at the total volume amounting to 0.1 ml of the preparation are done subcutaneously at three sites along the back in case of the first immunization, and into paw pads, in case of the second immunization.
3. To compromise the hematoencephalic barrier, one day before the first immunization and two days after it the mice are additionally intraperitoneally injected with 400 ng of pertussis toxin preparation.
4. Seventeen days after the first immunization, the mice receive a boosting peritoneal injection of the antigen in PBS at the total volume amounting to 0.2 ml, the dose of the immunogenic protein being 50 µg per mouse.
5. Concurrently with the boosting procedure, blood is taken from the orbital sinus of experimental and control (non-immunized) mice to monitor the development of the immune response. The presence of specific antibodies in the serum is determined by enzyme immunoassay.
6. Twenty one days after the beginning of the experiment, the mice that have showed the maximal antigen-specific response in immunochemical testing are used for splenectomy and blood sampling. The spleens are used for cell fusion in order to obtain hybridoma clones and for mRNA isolation for the subsequent cloning as phage display libraries.

To confirm the appearance of antigen-specific proteolytic antibodies in the course of immunization performed against the background of induced autoimmune pathology manifested as preclinical experimental autoimmune encephalomyelitis, the following tests are carried out:

### Analysis of the antigen specificity and proteolytic activity of the obtained polyclonal antibody preparations

### 1. Analysis of the antigen specificity of the obtained antibody preparations.

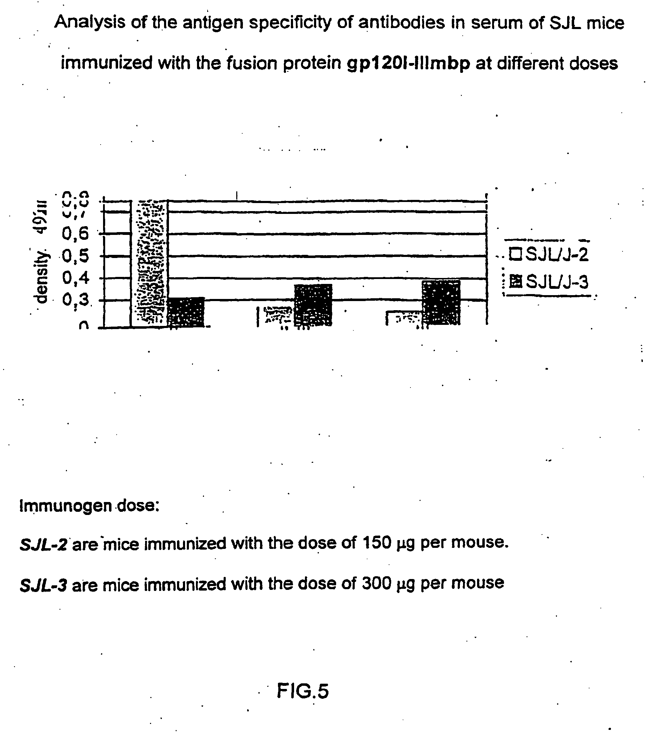

For the initial comparative monitoring of the specific immune response to the antigen in several immunized mice, enzyme immunoassay (EIA) is used (FIG. 5).

An antigen is immobilized on an immunological plate and, after incubation with sera obtained from immunized and control mice, antigen-antibody complex is detected with Fc-specific rabbit antimouse-IgG antibodies conjugated to horse radish peroxidase. The sera of immunized and control mice are used at several dilutions (1:12 and 1:48). The following recombinant proteins are used as antigens:
1) **trx-gp120 I-III-CH,** a fusion protein comprising *E. coli* thioredoxin A, a gp120 sequence lacking the first, second and third variable regions, and His₆-c-myc sequence;
2) **trx-gp120 I-II-H,** a fusion protein comprising *E*. *coli* thioredoxin A, the sequences of the first and second constant regions of gp120, and His₆ sequence;
3) **trx-gp120 II-III-H,** a fusion protein comprising *E*. *coli* thioredoxin A, the sequence of the second constant regions of gp 120, with the C-terminus of the sequence starting from the third constant region, and His₆ sequence;
4) **trx-gp120 III-H,** a fusion protein comprising *E*. *coli* thioredoxin A, the C-terminus of the gp120 sequence starting from the third constant region, and His₆ sequence; and
5) **trx-CH,** a fusion protein comprising *E*. *coli* thioredoxin A and His₆ sequence, which was used as the negative control to determine the non-specific binding of obtained antibodies to these protein sequences.

As a result of the performed experiments (FIG. 5), it has been shown that all the antibody preparations obtained from the serum of mice immunized with a fusion protein interact with the antigen.

### 2) Analysis of the proteolytic activity of the obtained polyclonal antibody preparations.

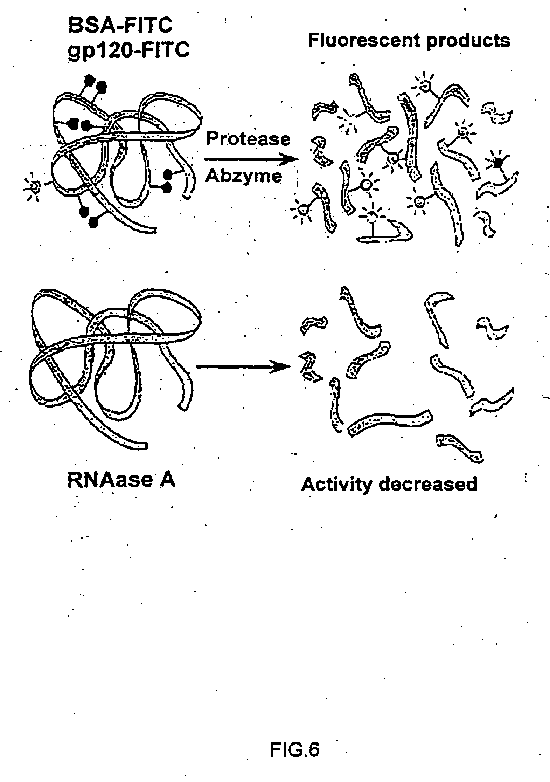

To determine the proteolytic activity, the antibodies are, as a preliminary, purified by affinity chromatography using recombinant protein G immobilized on Sepharose. The activity is detected by two different methods (FIG. 6).

A. Fluorescent assay. The principle of the method, which is outlined in FIG. 6A, is based on the phenomenon of fluorescence quenching by a protein heavily labeled with a fluorophore, which phenomenon is described in literature and is mainly based on the mutual interactions of the aromatic rings of different fluorophore molecules (e.g., because of intense hydrophobic and stacking contacts), and on fluorescence enhancement by introduction of breaks into the polypeptide chain. In the present test, bovine serum albumin and the recombinant protein **trx-gp120 I-III-CH** excessively labeled with fluorescein isothiocyanate (designated hereinafter as **BSA-FITC** and **gp120-FITC**) were used as the substrates for the proteolysis. The reaction was monitored by the fluorescence enhancement vs. control. Trypsin devoid of contaminating chymotrypsin activity was used as the model protease to determine the sensitivity of the method and to evaluate temporal signal changes depending on the substrate and enzyme amounts.

To determine the proteolytic activity of the tested antibody preparations, triplicate measurements are carried out at the baseline and after incubation at 37°C for 24 h and 48 h. The results of these experiments suggest the following (FIG.s 7-9):
- First, the proteolytic activity of preparations obtained from mice immunized with the protein **gp120 I-III-mbp** is increased in comparison with control ***SJL*** mice when both substrates are used.
- Second, the increase in the antigen-specific proteolytic activity is predominantly responsible for the total increase. With gp 120-FITC, the signal increased from ten to twenty times in comparison with non-immunized mice, whereas with BSA-FITC, the signal increased twofold only.
- Third, the predominant mechanism is, in this case, the serine-dependent catalysis, because the addition of serine-reactive irreversible inhibitors resulted in a significant reduction in the observed rate of hydrolysis of BSA-FITC.
- Forth, IgG molecules, which are selectively removed from the reaction by immunoprecipitation, are responsible for, at least, a major part of the observed proteolytic activity.

Along with undisputed advantages, such as high sensitivity and the simplicity and rapidity of measurements, this method, unfortunately, has some drawbacks, the main of which is complex nonlinear relationships between fluorescence changes and substrate and enzyme amounts, sample volume, buffer composition and pH, etc. resulting in difficulties in the quantitative evaluation and characterization of the enzymatic activity of polyclonal antibody preparations. Besides that, proteolytic activity determination by this test requires a large excess of substrate vs. enzyme, because the real amount of abzymes in the total pool of antibodies may make a few percents or less.

With regard to the above, another method for determination of the proteolytic activity of the obtained antibody preparations was used as an alternative.

### B. Enzymatic assay.

The principle of this method of detection of proteolytic activity, which is outlined in FIG. 6B, is based on the use of small amounts (about 1 ng per reaction) of a highly active enzyme ribonuclease A as the substrate of the proteolysis reaction. The level of ribonuclease activity, which linearly depends on the concentration of active RNAase A, is determined by the acid-soluble residue method using polycytidyl acid as the polymeric substrate of the reaction. This method of proteolytic activity detection presumably allows achieving a significant molar excess of enzyme vs. substrate and thus makes the conditions of the proteolysis reaction under study closer to those of the well-studied non-stationary kinetics model ([S]₀<<[E]).

To eliminate possible artefacts, all the antibody preparations under study were tested for the intrinsic ribonuclease activity and the presence of solution components that nonenzymatically alter the added ribonuclease activity. All the antibody preparations under study were devoid of the intrinsic ribonuclease activity and did not alter the added ribonuclease activity upon a short-time (10 min) incubation of the reaction.

The proteolytic activity of the tested antibody preparations was measured under the following conditions: IgG concentration 0.1 mg/ml, incubation time 17 h, temperature 37°C. The preparation activity measured by this method was expressed as ribonuclease hydrolysis rate.

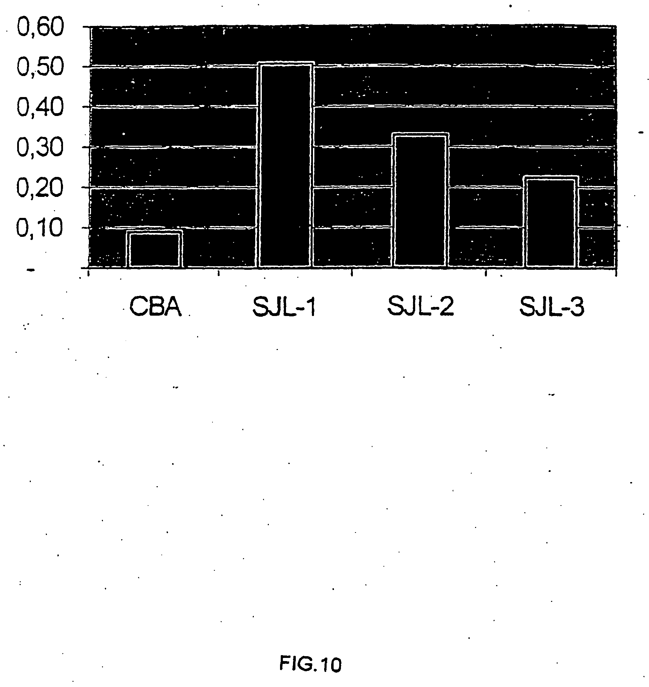

The test results presented in FIG. 10 show that proteolytic activity decreased 1.5-2 times upon immunization of mice with gp120-I-III-mbp. These results are in a limited correspondence with the results of the fluorescent test: only the negative correlation between the proteolytic activity and the immunogen dose used for immunization is reproduced. The discrepancy between the results of the fluorescent and enzymatic analysis might be explained by differences between the structures of the substrates of proteolysis. Presumably, RNAase A globule compared with BSA globule has fewer sites available for proteases and abzymes. Since, upon immunization, the total serum concentration of IgG increases manifold by antigen-specific antibodies, the proportion of the initial proteolytic antibodies decreases, whereas the newly-formed antigen-specific proteolytic antibodies are, most likely, inefficient catalyzers of proteolytic cleavage of RNAase A

### 3. Monitoring of immune response development and experimental autoimmune encephalomyelitis induction.

To characterize the features of immune response development upon immunization with a fusion protein comprising the neuroantigen MBP, a comparative analysis of specific surface markers expression in T-lymphocytes from SJL mice that were not immunized (control), from mice immunized with the synthetic peptide MBP₈₉₋₁₀₄, recombinant fusion protein gp120I-IIImbp at two different doses and recombinant protein gp120I-III was carried out (FIG. 11). All the immunizations were performed in parallel under identical conditions as described above. Twenty one days after the beginning of an experiment, CD4+ T-lymphocytes isolated from two mice of each group were analyzed by flow cytometry. A specific feature of SJL mice was initially low CD8+ T-cell counts. Also, changes in the expression of the following surface markers important for immune response development have been studied: CD11a, CD44, CD45RB, and CD62-L. The results presented in FIG. 11 suggest that in case of immunization with the peptide MBP and, also, with fusion proteins comprising this antigen, a fully developed T-cell immune response (memory cells appeared) was induced in mice by day 21 after the immunization, whereas in case of use of solely gp120I-III as the antigen, the immune response was still developing. The obtained data provide an evidence of the enhancement of immune response development when the autoantigen MBP is used and of T-lymphocyte activation typical of experimental autoimmune encephalomyelitis development.

Thus, the above Example shows that, upon immunization of SJL mice with the fusion protein **gp120I-IIImbp**, antigen-specific proteolytic antibodies are generated against the background of the preclinical stage of induced experimental autoimmune encephalomyelitis.

### Example 4. Synthesis of reactive phosphonate derivative of peptide fragment of gp120.

At the first stage, aminoalkylphosphonates protected at their free amino group are synthesized in the reaction of co-condensation of triphenylphosphite, isobutanal, and benzylcarbamate. To this end, the mixture of triphenylphosphite, isobutanal, and benzylcarbamate, 0.1 mole each, in 15 ml of glacial acetic acid is stirred for about 1 h until heat generation discontinues. After that, the reaction is stirred with heating to 80°C for 1 h. After the full completion of the reaction, volatile products are removed with a rotary evaporator under reduced pressure and heating on a water bath. The oily residue is dissolved in methanol (180 ml) and left for crystallization at -20°C for 3 h. After crystallization, the residue of diphenyl 1-(N-benzyloxycarbonyl)-aminoalkylphosphonate is harvested by filtration and recrystallized in a minimal volume of chloroform (30-40 ml) followed by the addition of four volumes of methanol.

To obtain free amynoalkylphosphonate, the protective group is removed by treatment of diphenyl 1-(N-benzylcarbonyl)-aminoalkylphosphonate with a 33% solution of hydrogen bromide in acetic acid (15 ml per 0.1 mole) for 1 h at room temperature. Volatile components are removed with a rotary evaporator at a reduced pressure and heating on a water bath. 1-(N-benzyloxycarbonyl)-aminoakylphosphonate hydrobromide is crystallized from the resulting residue by addition of anhydrous diethyl ether. Free phosphonate is obtained by passing gaseous dry ammonium through phosphonate hydrobromide suspension in diethyl ether until the formation of a thick precipitate of ammonium bromide discontinues and the full blooming of the suspension is observed. The resulting ammonium bromide is removed by filtration, and diethyl ether is evaporated on a water bath under atmospheric pressure.

To obtain the compound Leu-Ala-Glu-Glu-Glu-Val-*^{P}*(OPh)₂ (LAEEEV-*^{P}*(OPh)₂), where *^{P}*(OPh)₂ means the substitution of the α-carboxylic group with diphenylphosphonate, the peptide Boc-Val-Ala-(t-Bu)Glu-(t-Bu)Glu-(t-Bu)Glu protected at its N-terminal amino group and side groups is first synthesized. The peptide Boc-Val-Ala-(t-Bu)Glu-(t-Bu)Glu-(t-Bu)Glu is fused with the phosphonate derivative of valine by mixing of 2 µmoles of the protected peptide, 2 µmoles of the phosphonate, and 2 µmoles of dicyclohexylcarbodiimide in 300 µl of acetonitrile and incubating for 1 h. After the completion of the reaction, its products are separated by reverse phase HPLC on a 150×3.9-mm Waters C18 NovaPak column using 0% to 80% gradient of acetonitrile in 20 nM potassium phosphate (pH 7.0). The resulting fractions are analyzed by mass-spectrometry (MALDI-TOF). The fractions that contain substances with molecular ion masses of 1145 Da ([M+H]⁺), 1167 Da ([M+Na]⁺) or 1183 Da ([M+K]⁺) are combined and freeze-dried. The residue is dissolved in 100 µl of 100% trifluoroacetic acid and incubated for 1 h at room temperature to remove protective tert-butyloxycarbonyl and tert-butyl groups. The deblocked peptidylphosphonate is precipitated by addition of 10 volumes of anhydrous diethyl ether to the reaction. The precipitate is separated by centrifuging for 10 min at 12500 rpm, and the deblocking procedure is repeated. The residue is air-dried and stored at -20°C.

### Analysis of the peptidylphosphonate LAEEEV-^{p}(OPh)₂

### I. TOF MALDI mass-spectrometry.

1. A minimal amount of dry peptidylphosphonate, to which 5 µl of acetonitrile is added, is applied to a base plate using aqueous strong acid-free 2,5-dihytroxybenzoic (DHB) acid as the matrix, and analysis is carried out.
2. A TOF MALDI mass-spectrometer equivalent to the VISION 2000 apparatus is precalibrated with reference standards within the 500-2000 Da m/z range, and mass spectra of test samples are obtained using internal calibration. Molecular ion masses are determined using VISION 2000 Mass Analyzer software with the calibration taken into account. The expected result of the analysis is the presence of peaks corresponding to masses of 877.36, 900.34, and 915.45±1Da

### II. Analytical reverse phase HPLC.

1. To a 0.2 mg sample of peptidylphosphonate, 100 µl of 20 mM potassium phosphate buffer (pH 7.0) containing 20% acetonitrile is added.
2. The resulting sample is administered into an injector, and gradient elution is carried out using a 150x3.9 mm Waters C18 NovaPak column for reverse phase HPLC under the following conditions:
   - buffer A is 20% acetonitrile and 20 mM potassium phosphate, pH 7.0;
   - buffer B is 80% acetonitrile and 20 mM potassium phosphate, pH 7.0;
   - the elution rate is 1.0 ml/min at a linear 100% A to 100% B gradient for 20 min followed by 100% B for 10 min; and
   - the chromatograms are recorded at 260 nm wavelength.
3. The peaks are integrated without correction for the baseline. The retention time of the main peak, which has the maximal area, is determined, and the ratio of the main peak area to the sum of all the peak areas is calculated. The expected result: the retention time is 14.75-15.25 min; the chromatographic purity is >95%.

### III. Inhibition of the esterolytic activity of chymotrypsin.

1. 0.5 ml of 1 µM solution of α-chymotrypsin in a buffer containing 0.1 HEPES and 0.5 M NaCl, pH 7.2, is prepared. The solution is divided into nine 50-µl aliquots, and the residue is discarded.
2. Eight dilutions of the test peptidylphosphonate in acetonitrile ranging from 1000 µl to I µl are prepared.
3. To each of the first eight aliquots of the enzyme solution 5 µl of corresponding peptidylphosphonate solution are added, and 5 µl of acetonitrile are added to the ninth aliquot.
4. The samples are incubated for 1 h at 25±5°C.
5. The samples are successively transferred to a spectrophotometer cell containing 450 µl of deionized water, mixed, whereupon 10 µl of p-nitrophenylacetate solution in
   methanol (2.5 mg/ml) are added, after which the increase in the optical density at a 400-nm wavelength is recorded. The initial rate of the substrate hydrolysis is calculated in arbitrary units.
6. The effective inhibitor concentration is calculated. To this end, the ratios of the hydrolysis rates observed with samples 1-8 to the hydrolysis rate observed with sample 9 are calculated. The effective inhibitor concentration is determined as the lowest concentration of the test substance, at which the ratio of the rates of substrate hydrolysis does not exceed 50%. The expected result: 30 µM.

### Example 5. Reactive immunization of mice with the phosphonate derivative of a peptide fragment of gp120.

For immunization, the reactive peptide is conjugated to the macromolecular carrier *C*.*conholepas* hemocyanin (keyhole limpet hemocyanin, KLH). At the first stage, the carrier is activated with excess bis(sulfosuccinimidylyl)suberate in PBS for 1 h at 37°C. After the activation, KLH unbound to bis(sulfosuccinimidylyl)suberate is removed from the reaction by sevenfold exhaustive ultrafiltration (with the residual volume not more than 70 µl) using a Microcon 100 concentrator (Amicon YC membrane), each time adding PBS to the residue to make 500 µl and discarding the ultrafiltrate. To the resulting solution peptidylsulfonate solution in PBS is added whereupon the solution is incubated for 1 h at 37°C without stirring. The unreacted succinimide groups are inactivated by addition of 2 µl of 2-ethanolamine. The low molecular components of the reaction are removed by sevenfold exhaustive ultrafiltration (with the residual volume not more than 70 µl) using a Microcon 100 concentrator (Amicon YC membrane), each time adding PBS to the residue to make 500 µl and discarding the ultrafiltrate. The final preparation is sterilized by filtration and stored at -20°C

Female *MRL-lprllpr, **SJL*** and *NZB*/*NZW F₁* mice aged 6-8 weeks are intraperitoneally immunized with the antigen in complete Freund adjuvant, with the total volume being 0.2 ml, at the dose of 50 µg of the immunogenic protein per mouse.

The second immunization is done with the same volume and at the same antigen concentration in incomplete Freund adjuvant in 17 days afeter the first immunization. Concurrently, blood is withdrawn from the orbital sinus of three mice of each experimental group and control non-immunized mice of the three strains to monitor the development of the immune response.

Twenty one days after the beginning of the experiment, the mice that showed the maximal antigen-specific response in immunological tests are sacrificed for splenectomy. Polyclonal antibodies isolated from the blood serum of these mice are analyzed for antigen specificity and catalytic activity.

A part of the peptidylphosphonate synthesized at the previous stages is used in the reaction of conjugation to N-hydroxysuccinimide ester of biotin. The reaction is conducted by mixing of equimolar amounts of peptidyl sulfonate and activated biotin in a minimal volume of dimethylformamide and incubation for 1 h. The biotinylated preparations are intended for analysis of the specificity of the antibodies obtained as a result of reactive immunization of mice.

To monitor the specific immune response to the antigen in several immunized mice of all the three strains, enzyme immunoassay is used.

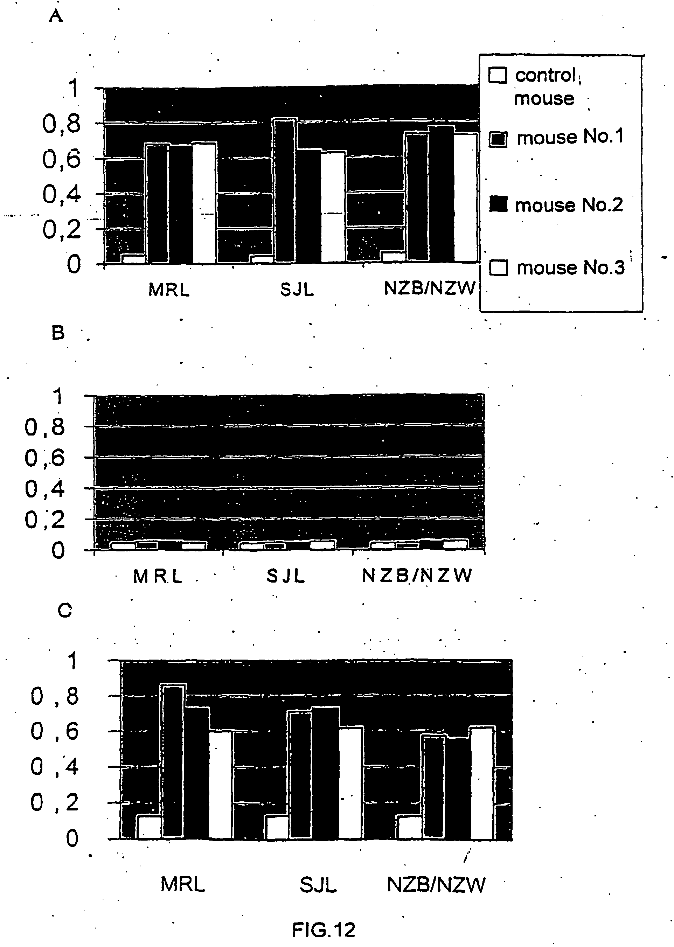

Antibodies from the sera of immunized and control mice are isolated with plate-preabsorbed goat antibodies against murine IgG, with subsequent incubation with the biotinylated antigen and detection of antigen-antibody complexes using neutravidin conjugated to horse radish peroxidase. The sera of immunized and control mice are used at several dilutions (1:12 and 1:48). The antigens employed are biotin-labeled *starting peptidylphosphonate*, biotinylated ***Val**-phosphonate*, and *nitrophenylmethyl-p-biotinylphenylmethylphosphonate*, for which the specific covalent modification of the active center of abzymes was demonstrated earlier. The comparative analysis has shown (FIG. 12) that the antibodies of the experimental mice of all the three strains, on the whole, possess a high specificity toward the modified peptide fragment of an antigen, do not interact under the conditions of the present experiment with free *val-phosphonate,* and exhibit the ability to covalently bind to a more active and less specific modifying agent. It should be noted that, on the average, in New Zealand hybrids the amount of antigen-specific antibodies was somewhat higher in comparison with the two other autoimmune strains, whereas the antibodies of ***MRL-lpr*/*lpr*** mice where more effective with regard to covalent modification.

Along with an antigen, horse radish peroxidase-conjugated rabbit antibodies against the Fc fragment of murine IgG are used to determine the total amount of murine antibodies specifically absorbed in a plate well. This allows estimation of the proportion of antigen-specific antibodies in the total pool of class G immunoglobulins.

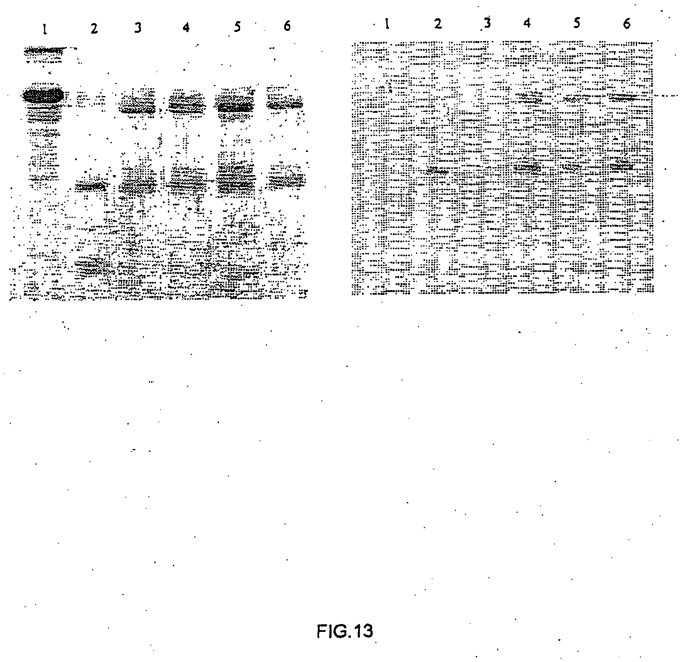

Further studies of the type of interaction of the obtained antibodies with a reactive peptide were carried out with pre-purified IgG preparations using immunoblotting. After incubation with biotinylated peptidylphosphonate, electrophoretic fractionation under denaturing and reducing conditions, and membrane immobilization, antigen-antibody complexes were detected using neutravidin conjugated to horse radish peroxidase. The results of this experiment presented in FIG. 13 suggest that both light and heavy immunoglobulin chains capable of being covalently modified by the peptide were present in the preparations of polyclonal antibodies isolated from autoimmune mice immunized with the reactive peptide ***Val-Ala-Glu-Glu-Glu-Val-PO(OPh)₂**.*

Thus, the reactive immunization under the conditions of the present Example has produced the following results:
- The antibodies obtained in the course of immunization bind to immunization antigen.
- The antibodies do not react with the "minimal" phosphonate group of immunization antigen, which means that there is no nonspecific interaction (or nonspecific chemical reaction) between the antibodies under study and the free phosphonate group of Val*^{P}*(OPh)₂
- The antibodies react with the active "mechanism-dependent" phosphonate, i.e., display the ability to react with a molecule that has no apparent structural relation to immunization antigen but has the ability to form covalent complexes with hydrolases.
- The antibodies form covalent complexes with immunization antigen.

In combination, the above properties suggest that in the course of immunization with a peptidylphosphonate whose composition corresponds to **LAEEEV-*^{P}*(OPh)₂** epitope-specific catalytic antibodies are generated.

### Industrial applicability

The invention may be useful in medicinal industry for manufacturing drugs.
## Claims

1. Fusion protein which has the following structure:

## Patentansprüche

1. Fusionsprotein das die folgende Struktur hat:

## Revendications

1. Protéine de fusion ayant la structure suivante:
